# EUROPEAN PATENT APPLICATION

(11) **EP 4 667 490 A1**
(43) Date of publication of application: **24.12.2025**
(21) Application number: 24305974.8
(22) Date of filing: 20.06.2024
(51) Int. Cl.: C07K 16/18, A61P 19/04

(54) **ANTI-PROCOLLAGEN ANTIBODIES AND USES THEREOF**

(71) Applicant: Université de Lorraine, 54000 Nancy (FR); Centre National de la Recherche Scientifique, 75016 Paris (FR)
(72) Inventor: VINCOURT, Jean-Baptiste, 54000 NANCY (FR)
(74) Representative: Plasseraud IP

(57) **Abstract**

The present invention pertains to an anti-procollagen antibody having the ability to inhibit the proteolytic cleavage of the procollagen C-terminal propeptide and/or the oxidative deamination of lysine residues in the tropocollagen C-telopeptide. Such an anti-procollagen antibody allows inhibiting procollagen maturation and can therefore advantageously be used for the treatment of diseases involving abnormal collagen synthesis and/or non-functional collagen synthesis. The antibody according to the present invention can particularly be used in the treatment of fibrosis, particularly idiopathic pulmonary fibrosis or liver fibrosis.

## Description

### Field of the Invention

The present invention pertains to novel therapeutic strategies for the treatment of fibrosis. The present invention particularly relates to an anti-procollagen antibody having the ability to inhibit the proteolytic cleavage of the procollagen C-terminal propeptide.

### Background of the Invention

Collagen is the main structural molecule found in the human body and represents the most abundant protein in mammals. Collagen is one of the main components of the extracellular matrix and can be found in all connective tissues such as cartilage, tendons, bones or dermis. It is an elongated fibrous protein made of the assembly of three polypeptide chains referred to as alpha-chains. Approximatively thirty different types of collagens have been identified to date; their nature and quantity depend on the type and function of the tissue in which there are found, but the vast majority of collagen found in the human body is type I collagen (representing over 90% of the total collagens comprised in the human body).

Defects in collagen synthesis can lead to serious conditions such as fibrosis. Fibrosis is a complication of several pathologies; it results from the massive synthesis and accumulation of non-functional collagen in a tissue, thereby affecting and impairing its function. Fibrosis can occur after repeated injuries, or after chronic or acute inflammation, and can affect several tissues/organs such as the liver, the joints, the heart and the lungs. Unfortunately, no treatment is available for treating fibrosis. The main aim of the therapeutic strategy is to relieve the symptoms as much as possible and slow down the progression of the disease.

Idiopathic pulmonary fibrosis (IPF) is a progressive, chronic and severe disease that affects the lungs and induces a progressive functional degeneration of the respiratory epithelium. It affects 500 in 100 000 people in developed countries with an average life expectancy of 5 years after diagnosis. The annual medical cost of IPF alone is more than 5 billion dollars in the US, and this amount is similar in Europe. The etiology of the disease remains unknown but is clearly multifactorial. Several studies have shown that scaring processes, involving collagen synthesis, following viral or bacterial pulmonary infections play an active role in the development and establishment of the disease. Preventing/reducing the synthesis of non-functional collagen after such infectious episodes thus represents a promising treatment pathway for IPF.

Several anti-fibrotic therapeutic approaches are currently being tested by the pharmaceutical industry. The most promising projects aim at interfering with the cellular pathways leading to the synthesis of collagen. Two drugs are currently approved for the treatment of IPF: Nintedanib and Pirfenidone. Nintedanib, sold by Boehringer Ingelheim, competitively inhibits both nonreceptor tyrosine kinases (nRTKs) and receptor tyrosine kinases (RTKs) thereby interfering with processes like fibroblast proliferation, differentiation and laying down extracellular matrix. Nintedanib was shown to improve IPF patients' quality of life, but does not improve survival and induces several adverse effects (in particular in the digestive tract). Pirfenidone, sold by Roche, is an anti-inflammatory and anti-fibrotic compound which acts via the downregulation of the production of growth factors and procollagens I and III. Intrapulmonary administration of Pirfenidone was shown to produce therapeutic responses in IPF patients with fewer side effects than when administered orally (Okano, Tomohito, et al. Frontiers in pharmacology 11 (2020): p. 1948).

Another approach for the treatment of fibrosis can be to target and inhibit BMP1 (bone morphogenetic protein 1), which is the major proteinase for C-terminal proteolysis of procollagen (N'Diaye, Elsa-Noah, et al. American Journal of Physiology-Cell Physiology (2020)). However, BMP1 was also shown to have numerous substrates other than collagen (Vadon-Le Goff, Sandrine et al. Matrix Biology 44 (2015): 14-23) and inhibiting this enzyme might thus induce several adverse effects. Furthermore, although BMP1 is the major proteinase for C-terminal proteolysis of procollagen, it is not the sole enzyme operating this cleavage (Broder, Claudia, et al. Proceedings of the National Academy of Sciences 110.35 (2013): 14219-14224). Inhibiting BMP1 alone would thus most likely not be sufficient for efficiently reducing the accumulation of non-functional collagen during fibrosis.

Another existing strategy is to directly inhibit the fibrotic process using antibodies that sterically block collagen fibrillogenesis (Steplewski et al. Fibrogenesis Tissue Repair 5, S29 (2012); Fertala et al. Connective tissue research 54.3 (2013): 187-196; Fertala et al. Connective tissue research 55.2 (2014): 115-122.). However, such a strategy requires using antibodies that target epitopes remaining in mature collagen, and therefore, these also bind assembled collagen, which is extremely abundant in the whole body and titrates therapeutic antibodies. In addition, for the same reason, such strategies have potential side effects.

There is thus an urgent need for new promising therapeutics for the treatment of idiopathic pulmonary fibrosis and of fibrosis in general.

### Summary of the Invention

The invention is defined by the claims.

The present inventors have demonstrated that it is possible to significantly reduce collagen accumulation in human primary chondrocytes, in cartilage explants and in primary human fibroblasts, by inhibiting tropocollagen extracellular maturation, particularly the proteolytic cleavage of the procollagen C-terminal propeptide and/or the oxidative deamination of lysine residues in the tropocollagen C-telopeptide. Such an inhibition prevents the maturation of collagen and through this, both the fibrillar assembly of collagen resulting from procollagen cleavage and its crosslinking, via independent mechanisms. Therefore, it represents an extremely promising strategy for the treatment of fibrosis. The present inventors have specifically produced anti-procollagen antibodies targeting the C-terminal cleavage site of either type I, II or III procollagen. Binding of each antibody to the amino acids immediately C-terminal to the cleavage site restrains its accessibility to enzymes operating the cleavage of the C-propeptide. In addition, the antibodies targeting type I or type II procollagen also inhibit the oxidative deamination of the corresponding C-telopeptide lysine, thereby preventing collagen crosslinking. The anti-procollagen antibody according to the invention thus allows reducing fibrotic processes underlying the development and establishment of fibrosis. In addition, the present inventors have also demonstrated that such an anti-procollagen antibody induces an inhibition of known fibrosis-related genes as well as of inflammation-related genes.

Accordingly, in a first aspect, the present invention pertains to an anti-procollagen antibody, wherein said antibody inhibits the extracellular maturation of procollagen, particularly by inhibiting the proteolytic cleavage of the procollagen C-terminal propeptide and/or the oxidative deamination of lysine residues in the tropocollagen C-telopeptide. The antibody according to the present invention particularly binds to a specific peptide region comprising the procollagen C-terminal cleavage site and/or amino acids located in the immediate vicinity in the C-terminal side of the procollagen C-terminal cleavage site.

The antibody according to the present invention can be used for the treatment of fibrosis, such as idiopathic pulmonary fibrosis or liver fibrosis. When used for the treatment of pulmonary fibrosis, the antibody according to the invention can be administered intrapulmonary, by inhalation through the nasal tract or through the mouth in the form of a dry powder or via an aerosol. The antibody according to the present invention can alternatively be administered intravenously, subcutaneously or intramuscularly in the form of an injectable solution.

### Detailed Description

Collagen is a structural biological polymer, the elemental building block of which is a triple-helical protein. The primary amino acid sequence of collagen is glycine-X-Y, wherein X is occasionally proline and Y is often hydroxyproline. Around 30 types of collagens have been identified to date and are, respectively, the products of distinct genes. The most abundant are types I, II, III and V, termed fibrillar collagens, type I representing the vast majority of collagens present in the human and animal body (around 90% of the total collagen found in the body). Fibrillar collagens are made of the assembly of three alpha chains. Unlike other fibrillar collagens, type I is normally made of the assembly of two identical chains (alpha 1) and an additional chain that differs slightly in its chemical composition (alpha 2). Type I collagen can be found in almost all connective tissues and is the major component of the interstitial membrane. Type II collagen is mainly found in cartilage. Type III collagen is generally associated with type I collagen and plays a structural role in hollow organs such as blood vessels and internal organs.

These different types of fibrillar collagens are synthesized via the same general synthesis pathway but they undergo post-translational modifications that differ depending on the tissue in which they are synthesized. Collagen synthesis involves both intra- and extracellular pathways. The main synthesis steps are as follows (for reference, see Wu, Marlyn et al. "Biochemistry, Collagen Synthesis." (2018 - updated in September 2020)):

### Intracellular:

1/ Transcription of the genes for pro-alpha 1 and pro-alpha 2 chains in the nucleus;
2/ mRNAs are routed from the nucleus to the cytoplasm for translation on ribosomes along the rough endoplasmic reticulum. The peptide chains obtained therefrom are referred to as "preprocollagen" or as "pre-pro-peptide chains";
3/ pre-pro-peptide chains are then transferred to the endoplasmic reticulum for post-translational modification. Three major modifications are made to the pre-pro-peptide:
   - the signal peptide on the N-terminal is removed (the resulting chains are referred to as "pro-alpha chains"),
   - specific lysine and proline residues get additional hydroxyl groups added to them via hydroxylase enzymes which require vitamin C (ascorbic acid) as a cofactor, and
   - glycosylation of the specific hydroxyl groups on lysine occurs optionally,
4/ three of the hydroxylated and glycosylated pro-alpha-chains (two pro-alpha-1 and one pro-alpha-2 for type I collagen) assemble by twisting into a triple helix by zipper-like folding (3 left-handed helices twisted into a right-handed coil) to form procollagen.
5/ procollagen is then transferred to the Golgi apparatus for further modifications including N-propeptide cleavage by procollagen N-proteinases (Canty EG, Kadler KE. J Cell Sci. 2005 Apr 1;118(Pt 7): 1341-53) and assembled into secretory vesicles to proceed to the extracellular space.

### Extracellular:

6/ Enzymes known as procollagen C-proteinases perform C-propeptide cleavage and remove the C- terminal ends (i.e. remove the C- terminal propeptide) of the procollagen molecule (as discussed above, BMP1 is the major enzyme for the cleavage of the C-terminal propeptide): the molecule remaining after this step is referred to as "tropocollagen";

7/ Specific lysine and hydroxylysine residues in the N- and C-telopeptides (the non-helical ends of tropocollagen) are oxidatively deaminated by lysyl oxidase (a copper-dependent enzyme) to form the respective aldehydic reactive species;

8/ tropocollagen molecules assemble into fibrils which are stabilized by covalent crosslinking resulting from the reactive species generated by lysyl oxidase.

Steps 6/ and 7/ as shown above correspond to the extracellular maturation of procollagen.

### Antibody according to the invention

The antibody according to the present invention allows inhibiting the proteolytic cleavage of the procollagen C-terminal propeptide and/or the oxidative deamination of lysine residues in the tropocollagen C-telopeptide. The present antibody thus inhibits step 6/ and 7/ of collagen synthesis as disclosed above, and, as such, inhibits the extracellular maturation of procollagen.

Accordingly, in a first aspect, the present invention pertains to an anti-procollagen antibody, wherein said antibody inhibits the extracellular maturation of procollagen. The antibody according to the present invention particularly inhibits the proteolytic cleavage of the procollagen C-terminal propeptide and/or the oxidative deamination of lysine residues in the tropocollagen C-telopeptide. In other words, the antibody according to the present invention inhibits the extracellular maturation (or "processing") of the procollagen and tropocollagen C-terminal end.

"**Procollagen**" is the precursor of tropocollagen. It is a triple helix formed by the assembly of three hydroxylated and glycosylated pro-alpha-chains prolonged by C- and N-terminal extensions. "**PC-collagen**" is procollagen from which the N-terminal extension has been cleaved off. The structure of collagen and of procollagens have been extensively studied in the prior are (see e.g. K. Henriksen and M.A. Karsdal, "Type I collagen" in Biochemistry of Collagens, Laminins and Elastin (Second Edition), 2019; Patrick Garnerov and Pierre D. Delmas, "Bone Turnover Markers" in Encyclopedia of Endocrine Diseases, 2004 ; or Elena Makareeva and Sergey Leikin, "Collagen Structure, Folding and Function" in Osteogenesis Imperfecta, 2014) and are thus familiar to the skilled person.

### Type I procollagen:

Type I procollagen is made by the assembly of two type I pro-alpha-1 chains and one type I pro-alpha-2 chain.

The amino acid sequence of the human type I procollagen pro-alpha-1 chain is as follows (accessible under the reference "P02452" in the Uniprot database):

MFSFVDLRLLLLLAATALLTHGQEEGQVEGQDEDIPPITCVQNGLRYHDRDVWKPEP
CRICVCDNGKVLCDDVICDETKNCPGAEVPEGECCPVCPDGSESPTDQETTGVEGPK
GDTGPRGPRGPAGPPGRDGIPGQPGLPGPPGPPGPPGPPGLGGNFAPQLSYGYDEKST
GGISVPGPMGPSGPRGLPGPPGAPGPQGFQGPPGEPGEPGASGPMGPRGPPGPPGKNG
DDGEAGKPGRPGERGPPGPQGARGLPGTAGLPGMKGHRGFSGLDGAKGDAGPAGP
KGEPGSPGENGAPGQMGPRGLPGERGRPGAPGPAGARGNDGATGAAGPPGPTGPAG
PPGFPGAVGAKGEAGPQGPRGSEGPQGVRGEPGPPGPAGAAGPAGNPGADGQPGAK
GANGAPGIAGAPGFPGARGPSGPQGPGGPPGPKGNSGEPGAPGSKGDTGAKGEPGPV
GVQGPPGPAGEEGKRGARGEPGPTGLPGPPGERGGPGSRGFPGADGVAGPKGPAGER
GSPGPAGPKGSPGEAGRPGEAGLPGAKGLTGSPGSPGPDGKTGPPGPAGQDGRPGPP
GPPGARGQAGVMGFPGPKGAAGEPGKAGERGVPGPPGAVGPAGKDGEAGAQGPPG
PAGPAGERGEQGPAGSPGFQGLPGPAGPPGEAGKPGEQGVPGDLGAPGPSGARGERG
FPGERGVQGPPGPAGPRGANGAPGNDGAKGDAGAPGAPGSQGAPGLQGMPGERGA

AGLPGPKGDRGDAGPKGADGSPGKDGVRGLTGPIGPPGPAGAPGDKGESGPSGPAGP
TGARGAPGDRGEPGPPGPAGFAGPPGADGQPGAKGEPGDAGAKGDAGPPGPAGPAG
PPGPIGNVGAPGAKGARGSAGPPGATGFPGAAGRVGPPGPSGNAGPPGPPGPAGKEG
GKGPRGETGPAGRPGEVGPPGPPGPAGEKGSPGADGPAGAPGTPGPQGIAGQRGVVG
LPGQRGERGFPGLPGPSGEPGKQGPSGASGERGPPGPMGPPGLAGPPGESGREGAPGA
EGSPGRDGSPGAKGDRGETGPAGPPGAPGAPGAPGPVGPAGKSGDRGETGPAGPTGP
VGPVGARGPAGPQGPRGDKGETGEQGDRGIKGHRGFSGLQGPPGPPGSPGEQGPSGA
SGPAGPRGPPGSAGAPGKDGLNGLPGPIGPPGPRGRTGDAGPVGPPGPPGPPGPPGPPS
AGFDFSFLPQPPQEKAHDGGRYYRADDANVVRDRDLEVDTTLKSLSQQIENIRSPEG
SRKNPARTCRDLKMCHSDWKSGEYWIDPNQGCNLDAIKVFCNMETGETCVYPTQPS
VAQKNWYISKNPKDKRHVWFGESMTDGFQFEYGGQGSDPADVAIQLTFLRLMSTEA
SQNITYHCKNSVAYMDQQTGNLKKALLLQGSNEIEIRAEGNSRFTYSVTVDGCTSHT
GAWGKTVIEYKTTKTSRLPIIDVAPLDVGAPDQEFGFDVGPVCFL (SEQ ID No. 1).

Procollagen N-peptidases cleave type I pro-alpha-1 chain between the 161^{th} and 162^{th} amino acid residues of SEQ ID No. 1, i.e. between a proline and a glutamine. Procollagen C-peptidases cleave type I pro-alpha-1 chain between the 1218^{th} and 1219^{th} amino acid residues of SEQ ID No. 1, i.e. between the alanine and aspartic acid residues underlined in SEQ ID No. 1 as shown above.

The N- and C-terminal peptides released from procollagen under the action of procollagen N-peptidases and procollagen C-peptidases, respectively, correspond to "**procollagen propeptides**" (N-terminal propeptide or C-terminal propeptide depending on the end from which they were released).

The peptide downstream the C-terminal cleavage site (i.e. from the 1219^{th} to the 1464^{th} amino acid residues as shown in SEQ ID No. 1) thus corresponds to the C-terminal propeptide of type I procollagen alpha 1 chain.

The amino acid sequence of the human type I pro-alpha-2 chain is as follows (accessible under the reference "P08123" in the Uniprot database):


























 (SEQ ID No. 2).

Procollagen N-peptidases cleave type I pro-alpha-2 chain between the 79^{th} and 80^{th} amino acid residues of SEQ ID No. 2, i.e. between an alanine residue and a glutamine residue. Procollagen C-peptidases cleave type I pro-alpha-2 chain between the 1119^{th} and 1120^{th} amino acid residues of SEQ ID No. 2, i.e. between the alanine and aspartic acid residues underlined in the sequence SEQ ID No. 2 as shown above. The peptide downstream the C-terminal cleavage site (i.e. from the 1120^{th} to the 1366^{th} amino acid residues as shown in SEQ ID No. 2) thus corresponds to the C-terminal propeptide of type I pro-alpha-2 chain.

### Type II procollagen:

Type II procollagen is made by the assembly of three type II pro-alpha-1 chains.

The amino acid sequence of the human type II pro-alpha-1 chain is as follows (accessible under the reference "P02458" in the Uniprot database):



























 (SEQ ID No. 3).

Procollagen N-peptidases cleave type II pro-alpha-1 chain between the 181^{st} and 182^{nd} amino acid residues of SEQ ID No. 3, i.e. between an alanine residue and a glutamine residue. Procollagen C-peptidases cleave type II pro-alpha-1 chain between the 1241^{st} and 1242^{nd} amino acid residues of SEQ ID No. 3, i.e. between the alanine and aspartic acid residues underlined in the sequence SEQ ID No. 3 as shown above. The peptide downstream the C-terminal cleavage site (i.e. from the 1242^{nd} to the 1487^{th} amino acid residues as shown in SEQ ID No. 3) thus corresponds to the C-terminal propeptide of type II pro-alpha-1 chain.

### Type III procollagen:

Type III procollagen is made by the assembly of three type III pro-alpha-1 chains. The amino acid sequence of the human type III pro-alpha-1 chain is as follows (accessible under the reference "P02461" in the Uniprot database):


























 (SEQ ID No. 4).

Procollagen C-peptidases cleave type III pro-alpha-1 chain between the 1221^{st} and 1222^{nd} amino acid residues of SEQ ID No. 4, i.e. between the glycine and aspartic acid residues underlined in the sequence SEQ ID No. 4 as shown above. The peptide the C-terminal cleavage site (i.e. from the 1222^{nd} to the 1466^{th} amino acid residues as shown in SEQ ID No. 4) thus corresponds to the C-terminal propeptide of type III pro-alpha-1 chain.

The antibody according to the present invention "**inhibits the proteolytic cleavage of the procollagen C-terminal propeptide**". According to the present invention, the antibody can inhibit the proteolytic cleavage of the C-terminal propeptide of any of the pro-alpha-chains constituting procollagen. The present antibody thus prevents step 6/ of collagen synthesis as disclosed above and thus inhibits the release of the procollagen C-terminal propeptide in the extracellular medium. The skilled person knows several methods which allow determining whether an antibody inhibits the release of the procollagen C-terminal propeptide. Such a method is e.g. disclosed in the examples of the present application. The antibody can e.g. be added to a medium comprising procollagen and procollagen C-peptidases, and the resulting product be e.g. submitted to Western blot analyses to evaluate whether the cleaved procollagen C-propeptide is present. If the presence of free procollagen C-propeptide/of cleaved procollagen is significantly reduced in presence of the antibody (in comparison to a control wherein no antibody is added to the medium), then it means that said antibody inhibits the proteolytic cleavage of the procollagen C-terminal propeptide.

The antibody according to the present invention typically acts by hindering the access to the cleavage site for procollagen C-peptidases, e.g. via steric hindrance on the C-terminal procollagen cleavage site.

The present antibody also "**inhibits the oxidative deamination of lysine residues in the tropocollagen C-telopeptide**". Accordingly, the antibody according to the present invention can also prevent step 7/ of collagen synthesis as disclosed above and thus prevents the action of lysyl oxidase resulting in the formation of the aldehydic forms of lysine and hydroxylysine in the tropocollagen C-telopeptide. The skilled person knows several methods which allow determining whether an amino acid residue is deaminated. The skilled person can e.g. detect the presence of the aminated and deaminated forms of amino acids by submitting them to mass spectrometry analysis.

According to a specific embodiment, the epitope targeted by the antibody according to the present invention, i.e. the specific amino acid sequence to which the antibody specifically binds, comprises the procollagen C-terminal cleavage site and/or amino acids located in the immediate vicinity in the C-terminal side of the procollagen C-terminal cleavage site (i.e. amino acids located in the C-terminal propeptide). By "immediate vicinity" it is meant that the epitope targeted by the antibody comprises or consists of amino acids that are located within the 25, preferably within the 20 amino acids adjacent to the procollagen C-terminal cleavage site. The epitope targeted by the antibody according to the present invention is located in the immediate vicinity in the C-terminal side of the procollagen C-terminal cleavage site, and, as such, in the procollagen C-terminal propeptide, i.e. downstream the procollagen C-terminal cleavage site, particularly within the first 25 N-terminal amino acids of the procollagen C-terminal propeptide sequence.

Suitable epitopes according to the present invention appear in bold letters in the amino acid sequences shown above. The antibody according to the present invention thus advantageously binds to one of the epitopes identified in bold letters in the amino acid sequences as shown above.

The antibody according to the present invention advantageously inhibits the extracellular maturation of type I, II or III procollagen.

According to a specific embodiment, the antibody according to the present invention specifically binds to an epitope located in a pro-alpha-1 chain. According to this embodiment, the antibody according to the present invention thus inhibits the proteolytic cleavage of the pro-alpha-1 chain C-terminal propeptide and/or the oxidative deamination of lysine residues located in the tropocollagen alpha-1 chain C-terminal telopeptide.

According to another specific embodiment, the antibody according to the present invention inhibits the proteolytic cleavage of the type I procollagen C-terminal propeptide and/or the oxidative deamination of lysine residues located in the type I tropocollagen C-terminal telopeptide, i.e. binds to an epitope located in type I procollagen. According to this embodiment, the antibody according to the present invention thus inhibits the synthesis of type I collagen.

According to a preferred embodiment, the antibody according to the present invention inhibits the proteolytic cleavage of the C-terminal propeptide of the type I pro-alpha-1 chain and/or inhibits the oxidative deamination of lysine residues located in the type-I-tropocollagen alpha-1-chain C-terminal-telopeptide. According to this preferred embodiment, the antibody according to the present invention binds to an epitope located in the type I pro-alpha-1 chain. Such an antibody advantageously binds to an epitope which comprises the cleavage site as shown in SEQ ID No. 1, i.e. which comprises the 1218^{th} and/or the 1219^{th} amino acid residues of SEQ ID No. 1, or to an epitope comprising amino acid residues which are in the immediate vicinity in the C-terminal end of said cleavage site, i.e. to amino acids located in the 25 amino acid residues downstream the pro-alpha-1 C-terminal cleavage site. According to a particularly preferred embodiment, such an antibody specifically binds to amino acids 1219 to 1238 of the amino acid sequence as shown in SEQ ID No. 1, i.e. to an epitope having the amino acid sequence DDANVVRDRDLEVDTTLKSL (SEQ ID No. 5).

According to another preferred embodiment, the antibody according to the present invention inhibits the proteolytic cleavage of the C-terminal propeptide of the type III pro-alpha-1 chain and/or inhibits the oxidative deamination of lysine residues located in the type-III-tropocollagen alpha-1-chain C-terminal-telopeptide. According to this preferred embodiment, the antibody according to the present invention binds to an epitope located in the type III pro-alpha-1 chain. Such an antibody advantageously binds to an epitope which comprises the cleavage site as shown in SEQ ID No. 4, i.e. which comprises the 1221^{st} and/or the 1222^{nd} amino acid residues of SEQ ID No. 4, or to an epitope comprising amino acid residues which are in the immediate vicinity in the C-terminal end of said cleavage site, i.e. to amino acids located in the 25 amino acid residues downstream the pro-alpha-1 C-terminal cleavage site. According to a particularly preferred embodiment, such an antibody specifically binds to amino acids 1222 to 1242 of the amino acid sequence as shown in SEQ ID No. 4, i.e. to an epitope having the amino acid sequence DEPMDFKINTDEIMTSLKSVN (SEQ ID No. 6).

The "**antibody**" according to the invention can be a human, murine, primate, gallinaceous bird (such as chickens) or camel antibody. It may be a chimeric or humanised antibody. It may be an antibody of any isotype or subclass thereof, in particular an IgG, IgM, IgA, IgE or IgD or an antigen-binding fragment thereof. Human immunoglobulin light chains include two domains, a variable domain (VL) and a constant domain (CL). Immunoglobulin heavy chains include four domains, a variable domain (VH) and three constant domains (CH1, CH2 and CH3, collectively referred to as CH). The variable regions of both light (VL) and heavy (VH) chains determine binding recognition and specificity to the antigen. The constant region domains of the light (CL) and heavy (CH) chains confer properties such as antibody chain association, secretion, trans-placental mobility, complement binding, and binding to Fc receptors (FcR). The "**antigen-binding fragment**" of an antibody (or simply "antibody fragment"), as used herein, refers to full length or one or more fragments of an antibody that retain the ability to specifically bind to an antigen. It has been shown that the antigen-binding function of an antibody can be performed by fragments of a full-length antibody. Examples of binding fragments encompassed within the term "antigen-binding fragment" of an antibody include Fab fragment, a monovalent fragment consisting of the VL, VH, CL and CH1 domains; a F(ab)2 fragment, a Fd fragment consisting of the VH and CH1 domains; a Fv fragment consisting of the VL and VH domains of a single arm of an antibody. Furthermore, although the two domains of the Fv fragment, VL and VH, are coded for by separate genes, they can be joined, using recombinant methods, by a synthetic linker that enables them to be made as a single chain protein in which the VL and VH regions pair to form monovalent molecules (known as single chain Fv (scFv); see e.g., Bird et al., 1988 Science 242:423-426; and Huston et al., 1988 Proc. Natl. Acad. Sci. 85:5879-5883). ScFv have a more rapid blood clearance, lower retention times in nontarget tissue and a reduced immunogenicity as compared to their parent antibodies (see Ahmad, Zuhaida Asra et al. Clinical & developmental immunology vol. 2012 (2012): art 980250). Such single chain antibodies are also intended to be encompassed within the term "antigen-binding fragment" of an antibody.

Preferred antibodies according to the present invention are humanized monoclonal antibodies (mAbs). The term "monoclonal antibody" as used herein refers to a preparation of antibody molecules of single specificity. A monoclonal antibody displays a single binding specificity and affinity for a particular epitope.

The antibody according to the present invention "specifically binds" to its targeted epitope, meaning that it typically binds to its targeted epitope with an equilibrium dissociation constant (Kd) of less than 50 nM, particularly less than 25nM, preferably less than 10nM, for example as measured by SPR assay, for example using Biacore^{®}.

### Use of the antibody according to the present invention

The antibody according to the present invention represents an extremely promising therapeutic tool for the treatment of conditions or diseases involving abnormal collagen accumulation and non-functional collagen accumulation such as fibrosis.

Fibrosis results from excess deposition of extracellular matrix components, mainly collagen, in tissues. It occurs when the synthesis of new collagen by myofibroblasts exceeds the rate at which it is normally degraded, resulting in an increase of the total amount of collagen over time. Fibrosis significantly impairs correct tissue functioning and results in deleterious chronic inflammation reactions.

Fibrosis can affect a large number of organs, the most common forms being idiopathic pulmonary fibrosis, liver cirrhosis, systemic sclerosis, progressive kidney disease, and cardiovascular fibrosis. Most chronic fibrotic disorders have in common a persistent inflammation that sustains the production of growth factors, proteolytic enzymes, angiogenic factors and fibrogenic cytokines, which stimulate the deposition of connective tissue elements that progressively remodel and destroy normal tissue architecture (see Wynn, Thomas A. "Cellular and molecular mechanisms of fibrosis." The Journal of Pathology: A Journal of the Pathological Society of Great Britain and Ireland 214.2 (2008): 199-210). Extensive tissue remodelling and fibrosis can ultimately lead to organ failure and death.

Fibrosis is extremely difficult to reverse and substantial evidence show that no reversal is possible for advanced fibrosis. The main therapies aim at relieving the symptoms as much as possible and slow down the progression of the disease. One of the main goals set for treating fibrosis is inhibiting the accumulation of fibrogenic cells and/or preventing the deposition of extracellular matrix proteins, in particular collagen (Bataller, Ramón, and David A. Brenner. "Liver fibrosis." The Journal of clinical investigation 115.2 (2005): 209-218).

Inhibiting tropocollagen synthesis is considered as one of the most promising pathway for the treatment of fibrosis.

By inhibiting the cleavage of the procollagen C-terminal propeptide and/or the oxidative deamination of lysine residues in the tropocollagen C-telopeptide, the antibody according to the present invention inhibits tropocollagen maturation and directly affects collagen assembly, and indirectly affects fibrotic extracellular matrix synthesis, without affecting cell viability. The present antibody was further shown to inhibit the expression of pro-inflammatory cytokines and collagen after stimulation with TGFβ.

Therefore, the antibody according to the present invention allows targeting pathways which are known to be key targets in the treatment of fibrosis and allows inhibiting fibrotic pathways.

Accordingly, in a further aspect, the present invention pertains to an anti-procollagen antibody inhibiting the proteolytic cleavage of the procollagen C-terminal propeptide and/or the oxidative deamination of lysine residues in the tropocollagen C-telopeptide for use as a medicament.

The present invention particularly pertains to an anti-procollagen antibody inhibiting the proteolytic cleavage of the procollagen C-terminal propeptide and/or the oxidative deamination of lysine residues in the tropocollagen C-telopeptide for use in the treatment of fibrosis in a patient.

Several organs have been shown to be affected by fibrosis among which are the lungs, the liver, the kidneys, the heart and the vascular system, the eye, the skin, the pancreas, the intestines, the brain or bone marrow (Wynn, 2008). The antibody according to the present invention can be used for the treatment of any type of fibrosis.

According to a preferred embodiment, the antibody according to the present invention is used for the treatment of pulmonary fibrosis, particularly idiopathic pulmonary fibrosis.

Excessive type I collagen synthesis was shown in multiple fibrotic disorders, and particularly in idiopathic pulmonary fibrosis (Kleaveland, Kathryn R., et al. "Fibrocytes are not an essential source of type I collagen during lung fibrosis." The Journal of Immunology 193.10 (2014): 5229-5239). Thus, the antibody used for treating fibrosis advantageously targets type I procollagen.

According to another preferred embodiment, the antibody according to the present invention is used for the treatment of liver fibrosis.

Type III collagen synthesis was shown to play an active role in the development and establishment of liver fibrosis (see Bataller, Ramón, and David A. Brenner (2005) mentioned above). Thus, the antibody used for specifically treating liver fibrosis can advantageously target type III procollagen.

As used herein, the terms "treating" or treatment" relate to reversing, alleviating, inhibiting the progress of, or preventing the disorder or condition to which such term applies (in the present case fibrosis) or reversing, alleviating, inhibiting the progress of, or preventing one or more symptoms of the disorder or condition to which such term applies.

The antibody according to the present invention, together with one or more conventional adjuvants, carriers, or diluents may be placed into the form of a pharmaceutical composition and unit dosage. The pharmaceutical compositions and unit dosage forms may comprise conventional ingredients in conventional proportions, with or without additional active compounds or principles. The skilled person knows how to introduce an antibody in such a pharmaceutical composition/unit dosage.

The antibody according to the present invention can be administered by any suitable route of administration. For example, the antibody according to the invention can be administered by oral (including buccal and sublingual), nasal, topical, pulmonary or parenteral (including intramuscular, intra-arterial/intravenous, intrathecal, subcutaneous and intravenous) administration or in a form suitable for administration by inhalation or insufflation.

When used for the treatment of idiopathic pulmonary fibrosis, the antibody according to the present invention is advantageously administered intrapulmonary, e.g. via an aerosol. Suitable formulations for intrapulmonary applications have a particle size typically in the range of 0.1 to 500 microns and are administered by inhalation through the nasal tract or through the mouth in the form of a dry powder or via an aerosol. Intrapulmonary administration of antibodies was e.g. used in Guillon et al (see Guillon, Antoine, et al. "Exploring the fate of inhaled monoclonal antibody in the lung parenchyma by microdialysis." MAbs. Vol. 11. No. 2. Taylor & Francis, 2019).

Local intrapulmonary administration of the anti-procollagen antibody according to the present invention advantageously induces fewer side-effects than when administered via other routes of administration in the context of IPF, as it allows specifically targeting the organ in which abnormal collagen synthesis occur, without affecting other organs where collagen still plays an essential structural role and is essential for proper organ function.

In a further aspect, the present disclosure provides a method for treating fibrosis, comprising administering an effective therapeutic amount of an anti-procollagen antibody inhibiting the proteolytic cleavage of the procollagen C-terminal propeptide to a patient in need thereof.

The "patient" to be treated according to the present invention is a mammal, particularly a human.

By a "therapeutically effective amount" is meant a sufficient amount of antibody to treat and/or to prevent fibrosis.

### Method for producing antibodies of the invention

Antibodies of the invention may be produced by any technique known in the art, such as, without limitation, any chemical, biological, genetic or enzymatic technique, either alone or in combination.

Antibodies directed against one specific epitope can be raised according to known methods by administering said epitope to a host animal selected, e.g., from pigs, cows, horses, rabbits, goats, sheep, chickens and mice, among others. Various adjuvants known in the art can be used to enhance antibody production. Although antibodies useful in practicing the invention can be polyclonal, monoclonal antibodies are preferred. Monoclonal antibodies can be prepared and isolated using any technique that provides for the production of antibody molecules by continuous cell lines in culture. Techniques for production and isolation include but are not limited to the hybridoma technique originally described by Kohler and Milstein (1975); the human B-cell hybridoma technique (Cote et al., 1983); and the EBV-hybridoma technique (Cole et al. 1985).

According to a specific embodiment, the antibody according to the present invention can be humanized. Humanized antibodies can be prepared according to known techniques. "Humanized antibodies" are forms of non-human (e.g., rodent) chimeric antibodies that contain minimal sequence derived from non-human immunoglobulin. For the most part, humanized antibodies are human immunoglobulins (recipient antibody) in which residues from a hypervariable region (CDRs) of the recipient are replaced by residues from a hypervariable region of a non-human species (donor antibody) such as mouse, rat, rabbit or nonhuman primate having the desired specificity, affinity and capacity. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally also comprises at least a portion of an immunoglobulin constant region (Fc), typically that of a human immunoglobulin. Methods for making humanized antibodies are described, for example, by Winter (U.S. Pat. No. 5,225,539) and Boss (Celltech, U.S. Pat. No. 4,816,397).

The Fab can be obtained by treating an antibody with a protease, papain. Also, the Fab can be produced by inserting DNA encoding Fab of the antibody into a vector for prokaryotic expression system, or for eukaryotic expression system, and introducing the vector into a procaryote or eucaryote (as appropriate) to express the Fab. F(ab)2 can be obtained by treating an antibody with a protease, pepsin. ScFv can be produced by obtaining cDNA encoding the VH and VL domains of an antibody, constructing DNA encoding scFv, inserting the DNA into an expression vector for prokaryote, or an expression vector for eukaryote, and then introducing the expression vector into a prokaryote or eukaryote (as appropriate) to express the scFv. To generate a humanized scFv fragment, a well-known technology called CDR grafting may be used, which involves selecting the complementary determining regions (CDRs) from a donor scFv fragment, and grafting them onto a human scFv fragment framework of known three dimensional structure (see, e.g., WO98/45322; WO 87/02671; US5,859,205; US5,585,089; US4,816,567; EP0173494).

### Nucleic acid sequence encoding an antibody of the invention

A further embodiment of the invention relates to a nucleic acid sequence encoding the antibody according to the present invention. Typically, said nucleic acid is a DNA or RNA molecule, which may be included in any suitable vector, such as a plasmid, cosmid, episome, artificial chromosome, phage or a viral vector.

The terms "vector", "cloning vector" and "expression vector" mean the vehicle by which a DNA or RNA sequence (e.g. a foreign gene) can be introduced into a host cell, so as to transform the host and promote expression (e.g. transcription and translation) of the introduced sequence. So, a further object of the invention relates to a vector comprising a nucleic acid encoding the antibody according to the present invention. Such vectors may comprise regulatory elements, such as a promoter, enhancer, terminator and the like, to cause or direct expression of said antibody upon administration to a subject.

A further object of the present invention relates to a cell which has been transfected, infected or transformed by a nucleic acid and/or a vector encoding the antibody according to the present invention. The term "transformation" means the introduction of a "foreign" (i.e. extrinsic or extracellular) gene, DNA or RNA sequence to a host cell, so that the host cell will express the introduced gene or sequence to produce a desired substance, typically a protein or enzyme coded by the introduced gene or sequence. A host cell that receives and expresses introduced DNA or RNA bas been "transformed". The nucleic acids of the invention may be used to produce an antibody of the invention in a suitable expression system. The term "expression system" means a host cell and compatible vector under suitable conditions, e.g. for the expression of a protein coded for by foreign DNA carried by the vector and introduced to the host cell.

The present invention will now be illustrated by the following Examples. These examples do not limit the scope of the claims.

### Brief Description of the Figures

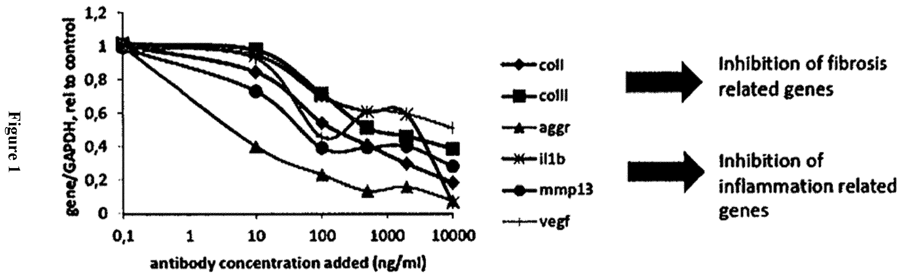

Figure 1: **mAb1** inhibits fibrosis- related genes and inflammation- related genes: Graph representing gene expression levels of several fibrosis- and inflammation-related genes (normalized to the expression of GADPH) in human primary chondrocytes cultures after treatment with mAb1.

### EXAMPLES

### - Material and Methods

**Antibody Synthesis** mAb1 is a monoclonal antibody targeting type II pro-alpha-1 chain. It binds to an epitope having the amino acid sequence DQAAGGLRQHDAEVDA, i.e. to amino acids 1242 to 1257 of SEQ ID no. 3 as shown above. Mice were immunized with a peptide comprising said epitope coupled to an N-terminal tail (consisting e.g. in lysine-leucine-histidine) and resulting T-lymphocytes clones were screened by ELISA so as to identify the target Mab-secreting clones.

**Cell culture.** Human articular cartilage was procured under general anesthesia during total knee replacements in the context of osteoarthritis. healthiest pieces of cartilage were cut off from articular surfaces, minced and rinsed in PBS several times over 1 h and pre-digested with 2 mg/ml pronase (Sigma) for 1 h at 37 °C in saline. Cartilage pieces were rinsed again and digested overnight with 1.5 mg/ml collagenase A (Roche) at 37 °C. The digest supernatant was filtered through 100 µm sterile filters (Millipore) and cells were collected after two centrifugations at 200 g for 5 min in serum-containing culture medium. Primary chondrocytes were inoculated at 1 million cells per plate into 6-well plates and grown to confluence in 15% FCS-supplemented DMEM/F12 (Gibco) containing 60 µg/ml ascorbate 2-phosphate under 2% oxygen pressure and maintained confluent for 2-3 days in Insulin, Transferrin, and Selenium (ITS, Sigma)-supplemented culture medium without serum. Where indicated, purified, PBS-dialysed antibody was added to the medium during the whole cultivation. All experiments were performed as technical triplicates. The antibody did not affect cell growth as judged by phase contrast microscopy. Depending on chondrocyte cultures, it did however modify cellular morphology.

**Explant culture.** Human articular cartilage was procured under general anesthesia during total knee replacements in the context of osteoarthritis. healthiest pieces of cartilage were cut off from articular surfaces, and explants were punched using 4 mm punchers. Explants were rinsed in PBS several times over 1 h and maintained in culture for 48 hours in FCS-free DMEM/F12 (Gibco) containing 60 µg/ml ascorbate 2-phosphate under 2% oxygen pressure. The medium was changed and purified, PBS-dialysed antibody was added to the medium for another 4 days. Media were harvested and precipitated for western blotting analysis.

**Western blotting.** At the end of cultivation, media were collected and 1 ml thereof was precipitated with DOC/TCA. Cell layers were rinsed in PBS, lysed in PBS, 1 % (v/v) triton X-100; insoluble proteins were retained after centrifugation, rinsed in water and further extracted in 0.5 M acetic acid (acid extract) for 3 days. Further extraction was performed in acetic acid containing 10 µg pepsin for the same duration (pepsin extract). Acid and pepsin extracts were neutralized and the collagen content was precipitated with DOC/TCA. All fractions were resuspended in SDS loading buffer; equivalent aliquots were loaded on SDS-PAGE, transferred to PVDF membranes and western blotting was performed using either the blocking anti-type II procollagen C-propeptide, an other in-house antibody directed against type I procollagen C-propeptide (PMID 20460531), or anti-COL2 antibody (Calbiochem) together with appropriate secondary HRP-conjugated antibodies. Blots were developed with the enhanced chemiluminescence system and acquired on a Chemidoc XRS+ imager (BIO-RAD).

**Gene expression analysis.** RNA was extracted from cell cultures using RNeasy kit (Qiagen). RT was performed from 0.5 µg total RNA using M-MLV reverse transcriptase (Invitrogen) with 250 ng random primers (Promega). Quantitative PCR was performed using 1/40 RT products described above using Power SYBR reagentmix in a StepOnePlus thermocycler (Applied Biosystems). Quantification was performed using standards from 10-4 to 101 pg target amplicon with maximal internal errors lower than 7 % and samples were compared only if processed together for all steps of the procedure for a given gene. GAPDH was used as internal standard. Primers used were: AGGR_FOR, TCTGTAACCCAGGCTCCAAC (SEQ ID No. 7); AGGR_REV, CTGGCAAAATCCCCACTAAA (SEQ ID No. 8); GAPDH FOR, CGACCACTTTGTCAAGCTCA (SEQ ID No. 9); GAPDH REV, AGGGGAGATTCAGTGTGGTG (SEQ ID No. 10); COL1_FOR, GGCCCAGAAGAACTGGTACA (SEQ ID No. 11); COL1_REV, CGCTGTTCTTGCAGTGGTAG (SEQ ID No. 12); COL2_FOR, ATGACAATCTGGCTCCCAAC (SEQ ID No. 13); COL2_REV, GAACCTGCTATTGCCCTCTG (SEQ ID No. 14); IL-1_REV, TCGTTATCCCATGTGTCGAA (SEQ ID No. 15); IL-1_FOR, GGACAAGCTGAGGAAGATGC (SEQ ID No. 16); MMP13 FOR, AATTGTCCGGTTTGTCTTGGA (SEQ ID No. 17); MMP13_REV, GGGAAGTGCTGGGGGATTTT (SEQ ID No. 18); VEGF_FOR, GCAGAATCATCACGAAGTGG (SEQ ID No. 19); VEGF_REV, GCATGGTGATGTTGGACTCC (SEQ ID No. 20). Absolute quantities of each gene were normalized to GAPDH and to untreated controls.

### - Results

Monoclonal antibody mAb1, which specifically targets type II procollagen was shown to specifically inhibit type II procollagen C-propeptide cleavage, oxidative deamination of lysine on the C-telopeptide and crosslinking in both chondrocyte cultures and tissue explants. mAb1 directly affects collagen assembly and fibrotic extracellular matrix (via cellular signalling), without affecting cell viability. Under stimulation with TGFβ, mAb1 strongly inhibits the expression of collagen and pro-inflammatory cytokine genes. Without being bound by theory, the present inventor submits that by inhibiting procollagen maturation, the antibody according to the present invention triggers a quality control signal which stops collagen synthesis and frees the cell from the burden of collagen production, thereby improving its homeostasis and inflammation healing.

The data obtained show that mAb1delays and partially blocks type II procollagen cleavage at a dose of 6 µg/mL, as it induces accumulation of cleaved type II procollagen C-propeptide and that of uncleaved procollagen and PC-collagen in the extracellular matrix (data not shown). In result, it reduces very significantly the accumulation of mature type II collagen in the extracellular matrix.

The cleaved C-propeptide of type II procollagen is capable of re-penetrating cells (PMID: 20460531). mAb1 significantly affects type II collagen synthesis and is correlated with a specific inhibition of type II collagen C-propeptide cellular penetration.

mAb1 inhibits fibrosis related genes and inflammation related genes (see Figure 1).

Finally, mAb1 retains its capacity to block type II collagen C-propeptide cleavage in dense tissues where chondrocytes have a pericellular environment close to the in vivo situation (cartilage explants).

The results shown above can be directly applied to a similar strategy targeting type I or III procollagen. Indeed, the present antibody inhibits the final 2 steps of procollagen maturation via steric hindrance on the C-terminal cleavage site and on the C-telopeptide lysine deamination. Procollagen maturation steps are highly conserved between collagens and particularly between types I, II and III. Type I and II collagens are interchangeably used as examples for studying mechanisms applicable to other types of collagens. Studies are generally carried out on Type I collagen, which is easily obtainable, and the results obtained therefrom are known to be generalizable to other collagen types. In the same manner, data obtained with respect to type II collagen can be applied to other collagen types such as type I or III collagen. The results shown above can thus be extended type I or III collagen when using an anti- type I procollagen antibody inhibiting the proteolytic cleavage of the type I procollagen C-terminal propeptide.

## Claims

1. An anti-procollagen antibody, wherein said antibody inhibits the extracellular maturation of procollagen.

2. The antibody according to claim 1, wherein said antibody inhibits the proteolytic cleavage of the procollagen C-terminal propeptide and/or the oxidative deamination of lysine residues in the tropocollagen C-telopeptide.

3. The antibody according to claim 1 or 2, wherein said antibody binds to an epitope comprising the procollagen C-terminal cleavage site and/or amino acids located in the immediate vicinity in the C-terminal side of the procollagen C-terminal cleavage site.

4. The antibody according to any one of claims 1 to 3, wherein said antibody binds to an epitope located in a pro-alpha-1 chain.

5. The antibody according to any one of claims 1 to 4, wherein said procollagen is type I procollagen.

6. The antibody according to any one of claims 1 to 5, wherein said antibody inhibits the proteolytic cleavage of the type I pro-alpha-1 chain C-terminal propeptide.

7. The antibody according to claim 6, wherein said antibody binds to an epitope having the amino acid sequence DDANVVRDRDLEVDTTLKSL (SEQ ID No. 5).

8. The antibody according to any one of claims 1 to 4, wherein said procollagen is type III procollagen, and said antibody binds to an epitope having the amino acid sequence DEPMDFKINTDEIMTSLKSVN (SEQ ID No. 6).

9. The antibody according to any one of claims 1 to 8, for use as a medicament.

10. The antibody according to any one of claims 1 to 8, for use in the treatment of fibrosis in a patient.

11. The antibody for use according to claim 10, wherein said fibrosis is a pulmonary fibrosis, preferably idiopathic pulmonary fibrosis.

12. The antibody for use according to claim 11, wherein said antibody is administered intrapulmonary, intravenously, subcutaneously or intramuscularly.

13. The antibody for use according to claim 10, wherein said fibrosis is liver fibrosis.

14. The antibody for use according to any one of claims 10 to 13, wherein said patient is a mammal, preferably a human.
